# EUROPÄISCHE PATENTANMELDUNG

(11) **EP 1 627 621 A1**
(43) Veröffentlichungstag der Anmeldung: **22.02.2006**
(21) Anmeldenummer: 05009954.8
(22) Anmeldetag: 06.05.2005
(51) Int. Cl.: A61K 8/02, A61Q 5/06, A61Q 17/04, A61K 8/81, A61K 8/87, A61K 8/73, A61K 8/49

(54) **Haarstylinggel mit Lichtschutzwirkung für Haut und Haare**

(30) Priorität: 15.07.2004 DE 102004034265
(71) Anmelder: Wella Aktiengesellschaft, 64274 Darmstadt (DE)
(72) Erfinder: Lauscher, Dirk, Dr., 64297 Darmstadt (DE); Hess, Gabriele, 64390 Erzhausen (DE); Birkel, Susanne, Dr., 64285 Darmstadt (DE); Thiel, Rolf, Dr., 64285 Darmstadt (DE); Franzke, Michael, Dr., 64380 Rossdorf (DE)

(57) **Zusammenfassung**

Es wird ein Haarstylingmittel in Gelform beschrieben mit einem Gehalt an einer Kombination aus mindestens einem Gelbildner, mehr als 2 Gew.% mindestens eines UV-Filters und mindestens einem haarfestigenden Polymer. Das Stylinggel liegt vorzugsweise in optisch klarer Form vor und kann gleichzeitig eine haarfestigende Wirkung, eine Lichtschutzwirkung für die Haut und eine Lichtschutzwirkung für die Haare oder die Haarfarbe ausüben. Bevorzugte UV-Filter weisen sowohl eine Benzimidazol-Struktureinheit als auch Sulfonsäuregruppen auf.

## Beschreibung

Gegenstand der Erfindung ist ein Haarstylinggel mit Lichtschutzwirkung für Haut und Haare mit einem Gehalt an Gelbildner, mehr als 2 Gew.% UV-Filter und mindestens einem haarfestigenden Polymer.

Haarbehandlungsmittel in Form von Gelen werden eingesetzt, um dem menschlichen Haar Festigung und Halt zu geben, um die Frisur zu stabilisieren oder um die Frisiereigenschaften oder die Haarstruktur zu verbessern. Bevorzugt werden dabei optisch klare oder zumindest nahezu optisch klare, durchscheinende Produkte. Wünschenswert sind Produkte, welche einen über die Grundwirkung hinausgehenden Zusatznutzen aufweisen, z.B. eine Sonnenschutzwirkung für die mit dem Produkt in Kontakt kommende Kopfhaut oder eine Lichtschutzwirkung für die behandelten Haare oder eine Lichtschutzwirkung für die Haarfarbe. Bekannt sind Haarstylinggele, welche zum Schutz der Produktmasse geringe Mengen an Lichtschutzstoffen enthalten. Die Mengen sind aber zu gering, um einen ausreichenden UV-Schutz für die Haut bewirken zu können. Derartige Haargele mit geringen Mengen an UV-Filtern sind z.B. beschrieben in WO 01/13884, US 5,843,415 oder EP 867 167. Eine Erhöhung der Einsatzmenge an UV-Filtern ist nicht ohne weiteres möglich, da zu erwarten ist, dass dies die Produktstabilität von klaren Gelen beeinträchtigen kann, insbesondere hinsichtlich Klarheit, Viskositätsverhalten, Konsistenz, unerwünschten Verfärbungen oder Inkompatibilitäten mit weiteren Inhaltstoffen. Viele hochwirksame UV-Filter sind z.B. hydrophober Natur. Die Verwendung von größeren Mengen hydrophober, wassserunlöslicher Komponenten in den üblicherweise wasserbasierten Stylinggelen kann die optische Klarheit des Produkts beeinträchtigen. Hydrophile UV-Filter können ionisch oder nichtionisch sein. Ionische Komponenten haben aufgrund ihres Elektrolytcharakters in der Regel einen negativen, viskositätsmindernden Einfluß auf die Viskosität der Gele, wenn sie in hohen Konzentrationen eingesetzt werden, da die üblicherweise in Stylinggelen eingesetzten Gelbildner häufig gegenüber Elektrolyten empfindlich sind. Außerdem sind Schwierigkeiten zu erwarten, wenn in ein Gel mit hoher Elektrolytbelastung hydrophobe Stoffe wie z.B. Parfümöle in optisch klarer Form einemulgiert werden sollen. Hydrophobe Parfümöle können in herkömmliche wässrige Gele mittels geeigneter Emulgatoren in optisch klarer Form eingearbeitet werden. Bei hoher Elektrolytbelastung ist das aber nicht ohne weiteres möglich und es kann zu Trübungen, Entmischungen oder Inhomogenitäten kommen. Nichtionische hydrophile UV-Filter, z.B. solche mit Polyethylenglyolketten, können bei längerer Lagerung und unter Lichteinfluß zu unerwünschten, gelblichen Verfärbungen führen, wenn sie in größeren Mengen in klaren Gelen eingesetzt werden.

Es bestand die Aufgabe, ein Haarstylinggel zur Verfügung zu stellen mit einem Dreifachnutzen, nämlich einer haarfestigenden Wirkung, einer Lichtschutzwirkung auf die Haut und einer Lichtschutzwirkung auf die Haare. Das Gel sollte idealerweise optisch klar oder zumindest nahezu klar oder durchscheinend sein und auch bei längerer Lagerung eine ausreichende Stabilität hinsichtlich Konsistenz und optischem Eindruck aufweisen.

Gegenstand der Erfindung ist ein Haarstylinggel mit einem Gehalt an einer Kombination von
(A) mindestens einem Gelbildner;
(B) mehr als 2 Gew.% mindestens eines UV-Filters und
(C) mindestens einem haarfestigenden Polymer.

Der Gelbildner (A) ist in dem erfindungsgemäßen Gel vorzugsweise in einer Menge von 0,1 bis 20 Gew.% oder von 0,5 bis 10 Gew.% enthalten. Die UV-Filter (B) sind vorzugsweise in einer Menge von bis zu 15 Gew.%, insbesondere von 3 bis 9 Gew.% enthalten. Das haarfestigende Polymer (C) ist vorzugsweise in einer Menge von 0,1 bis 15 Gew.%, 0,2 bis 10 Gew.% oder 0,5 bis 5 Gew.% enthalten.

Besonders bevorzugt sind optisch klare oder nahezu klare, leicht opake Produkte, insbesondere Gele, bei denen Trübungen mit bloßem Auge nicht erkennbar sind. Der Lichtschutzfaktor (LSF) des Gels ist vorzugsweise mindestens 4, insbesondere größer oder gleich 6, größer oder gleich 10 oder größer oder gleich 15. Der LSF kann bestimmt werden z.B. nach der in der COLIPA-Publikation "International Sun Protection Factor Test Method" (2003) beschriebenen standardisierten Methode.

### Gelbildner

Der Gelbildner (A) ist vorzugsweise ein gelbildendes, verdickendes Polymer, z.B.
- Copolymere aus mindestens einer ersten Monomerart, die ausgewählt ist aus Acrylsäure und Methacrylsäure und mindestens einer zweiten Monomerart, die ausgewählt ist aus Estern der Acrylsäure und ethoxyliertem Fettalkohol z.B. Acrylates/Palmeth-25 Acrylate Copolymer, Synthalen® 2000W;
- vernetzte Polyacrylsäure, z.B. Carbomere, Carbopol® 980, 981, 2001 ETD, 2020 ETD, Carbomer K;
- vernetzte Copolymere aus mindestens einer ersten Monomerart, die ausgewählt ist aus Acrylsäure und Methacrylsäure und mindestens einer zweiten Monomerart, die ausgewählt ist aus Estern der Acrylsäure mit C10- bis C30-Alkoholen; z.B. Acrylates/C10-30 Alkylacrylate Crosspolymer, Pemulen® TR1, Carbopol® Ultrez 21;
- Copolymeren aus mindestens einer ersten Monomerart, die ausgewählt ist aus Acrylsäure und Methacrylsäure und mindestens einer zweiten Monomerart, die ausgewählt ist aus Estern der Itaconsäure und ethoxyliertem Fettalkohol z.B. Acrylates/Steareth-20 Itaconate Copolymer, Acrylates/Ceteth-20 Itaconate Copolymer, Structure® 2001, Structure® 3001;
- Copolymeren aus mindestens einer ersten Monomerart, die ausgewählt ist aus Acrylsäure und Methacrylsäure, mindestens einer zweiten Monomerart, die ausgewählt ist aus Estern der Itaconsäure und ethoxyliertem C10- bis C30-Alkohol und einer dritten Monomerart, ausgewählt aus C1- bis C4-Aminoalkylacrylaten; z.B. Acrylates/Aminoacrylates/C10-30 Alkyl PEG-20 Itaconate Copolymer, Structure® Plus;
- Copolymeren aus zwei oder mehr Monomeren, ausgewählt aus Acrylsäure, Methacrylsäure, Acrylsäureestern und Methacrylsäureestern, z.B. Acrylates Copolymer, Carbopol® Aqua SF-1;
- Copolymeren aus Vinylpyrrolidon und Ammoniumacryloyldimethyltaurat; z.B. Aristoflex® AVC;
- Copolymeren aus Ammoniumacryloyldimethyltaurat und Monomeren ausgewählt aus Estern von Methacrylsäure und ethoxylierten Fettalkoholen z.B. Ammoniumacryloyldimethyltaurat/Beheneth-25 Methacrylat Copolymer, Aristoflex® HMB;
- Hydroxyalkylcellulosen, z.B. Hydroxyethylcellulose, Hydroxypropylcellulose, Natrosol® 250 HHR;
- Hydroxypropylguar;
- Glycerylpolyacrylat und Glycerylpolymethacrylat, z.B. Hispagel® 100, Hispagel® 200, Lubrajel®;
- Copolymeren aus mindestens einem C2-, C3- oder C4-Alkylen und Styrol;
- Verdickenden Polyurethanen;
- Hydroxypropylstärkephosphat, z.B. Structure® XL;
- Polyacrylamid, z.B. Sepigel® 305;
- mit Decadien vernetztes Copolymer aus Maleinsäureanhydrid und Methylvinylether, z.B. PVM/MA Decadiene Crosspolymer, Stabileze® 06;
- Copolymere aus Polyethylenoxid, Fettalkoholen und gesättigtem Methylendiphenyldiisocyanat (z.B. PEG-150/Stearylalkohol/ SMDI Copolymer);
- Verdicker auf natürlicher Basis wie z.B. Johannesbrotkernmehl; Guar-Gummi; Xanthan; Dehydroxanthan; Carrageenan; Karaya-Gummi; hydrolysierte Maisstärke.

Bevorzugte Gelbildner sind Acrylates/C10-30 Alkylacrylate Crosspolymere, Carbomere, Xanthangum und Acrylates/Ceteth-20 Itaconate Copolymer.

### UV-Filter

Die UV-Filter (B) sind vorzugsweise organische Verbindungen, welche Strahlung im Bereich der UV-A-Strahlung (315 - 400 nm) und/oder im Bereich der UV-B-Strahlung (280-315 nm) absorbieren. Bevorzugte UV-Filter weisen mindestens eine Säuregruppe, insbesondere mindestens eine -SO₃H-Gruppe auf und können in teilweise oder vollständig neutralisierter Form, d.h. als Salze eingesetzt werden bzw. im Endprodukt vorliegen. Salze sind z.B. die Ammonium-, Alkali- oder Erdalkalisalze, insbesondere die Natriumsalze. Bevorzugt sind auch UV-Filter, welche mindestens eine Benzimidazol- oder Benzoxazol-Einheit im Molekül aufweisen.

Sulfonsäuresubstituierte Benzimidazole und Benzoxazole haben z.B. die allgemeine Formel 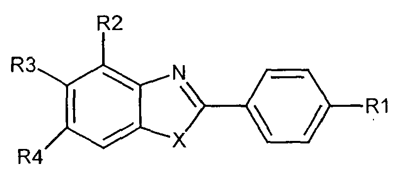
wobei R1 für Wasserstoff, eine C1- bis C10-Alkoxygruppe oder für die Gruppe 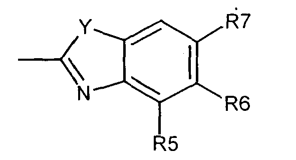
steht;
R2 bis R7 stehen unabhängig voneinander für Wasserstoff und für -SO₃H, wobei mindestens einer der Reste R2 bis R7 für - SO₃H steht;
X und Y stehen für Sauerstoff oder NH.

Besonders bevorzugt sind Verbindungen, bei denen X und Y (sofern vorhanden) gleich NH sind und wobei R2 und R4 sowie R5 und R7 jeweils gleich und verschieden von R3 bzw. R6 sind. Benzimidazole sind beispielsweise 2-Phenyl-1H-benzimidazol-5-sulfonsäure (z.B. Neo Heliopan® Hydro), 2,2'-(1,4-Phenylen)-bis-(1H-benzimidazol-4,6-disulfonsäure) (z.B. Neo Heliopan® AP) und 2,2'-(1,4-Phenylen)bis-(1H-benzimidazol-5-sulfonsäure) und deren jeweiligen Salze. Ein Benzoxazol ist z.B. 2,2'-(1,4-Phenylen)bis-(oxazol-5-sulfonsäure) und dessen Salze.

Vorzugsweise ist in dem erfindungsgmäßen Gel eine Kombination von mindestens einem UV-A-Filter mit mindestens einem UV-B-Filter enthalten, insbesondere eine Kombination von Phenylbenzimidazolsulfonsäure oder deren Salzen mit Phenyldibenzimidazoltetrasulfonsäure oder deren Salzen.

### Haarfestigende Polymere

Haarfestigende Polymere sind z.B. diejenigen Polymere, die im International Cosmetic Ingredient Dictionary and Handbook, 10. Auflage, 2004, Vol 3 unter der Funktion "Hair Fixatives" beschrieben sind. Haarfestigende Polymere sind insbesondere diejenigen, die bei Anwendung in 0,01 bis 5%igen Zubereitungen in einem geeigneten wässrigen, alkoholischen oder wässrig-alkoholischen Lösungsmittel in der Lage sind, eine haarfestigende Wirkung auszuüben, feststellbar z.B. durch übliche Curl Retention Messungen.

Haarfestigende Polymere können nichtionisch, anionisch, kationisch, zwitterionisch oder amphoter sein. Wenn einer der polymeren Gelbildner oder eines der haarfestigenden Polymere anionisch ist, ist vorzugsweise ein kationisches Polymer nicht oder nur in sehr geringen Mengen (z.B. kleiner 1 oder kleiner 0,1 Gew.%) enthalten, da es sonst zu unerwünschten Wechselwirkungen kommen kann.

Anionische Polymere sind Polymere mit anionischen oder anionisierbaren Gruppen. Unter anionisierbaren Gruppen werden Säuregruppen wie z.B. Carbonsäure-, Sulfonsäure- oder Phosphorsäuregruppen verstanden, welche mittels üblicher Basen wie z.B. organischer Amine oder Alkali- oder Erdalkalihydroxide deprotoniert werden können.

Polymere mit Säuregruppen können teilweise oder vollständig mit einem basischen Neutralisationsmittel neutralisiert sein. Bevorzugt sind solche Mittel, in welchen im Polymer die sauren Gruppen zu 50 bis 100 %, besonders bevorzugt zu 70-100% neutralisiert sind. Als Neutralisationsmittel können organische oder anorganische Basen verwendet werden. Beispiele für Basen sind insbesondere Aminoalkanole wie z.B. Aminomethylpropanol (AMP), Triethanolamin oder Monoethanolamin, aber auch Ammoniak, NaOH, KOH u.a..

Das anionische Polymer kann ein Homo- oder Copolymer mit Säuregruppen enthaltenden Monomereinheiten auf natürlicher oder synthetischer Basis sein, welches gegebenenfalls mit Comonomeren, die keine Säuregruppen enthalten, copolymerisiert ist. Als Säuregruppen kommen Sulfonsäure-, Phosphorsäure- und Carbonsäuregruppen in Betracht, von denen die Carbonsäuregruppen bevorzugt sind. Geeignete Säuregruppen enthaltende Monomere sind beispielsweise Acrylsäure, Methacrylsäure, Crotonsäure, Maleinsäure bzw. Maleinsäureanhydrid, Maleinsäuremonoester, insbesondere die Mono-C1-C7-alkylester der Maleinsäure sowie Aldehydocarbonsäuren oder Ketocarbonsäuren. Nicht mit Säuregruppen substituierte Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylester, Vinylalkohol, Propylenglykol oder Ethylenglykol, aminsubstituierte Vinylmonomere wie z.B. Dialkylaminoalkylacrylat, Dialkylaminoalkylmethacrylat, Monoalkylaminoalkylacrylat und Monoalkylaminoalkylmethacrylat, wobei die Alkylgruppen dieser Monomere vorzugsweise C1- bis C7-Alkylgruppen, besonders bevorzugt C1- bis C3-Alkylgruppen sind.

Geeignete Polymere mit Säuregruppen sind insbesondere unvernetzte oder mit polyfunktionellen Agenzien vernetzte Homopolymere der Acrylsäure oder der Methacrylsäure, Copolymere der Acrylsäure oder Methacrylsäure mit Monomeren ausgewählt aus Acrylsäure- oder Methacrylsäureestern, Acrylamiden, Methacrylamiden und Vinylpyrrolidon, Homopolymere der Crotonsäure sowie Copolymere der Crotonsäure mit Monomeren ausgewählt aus Vinylestern, Acrylsäure- oder Methacrylsäureestern, Acrylamiden und Methacrylamiden. Ein geeignetes natürliches Polymer ist beispielsweise Schellack.

### Bevorzugte Polymere mit Säuregruppen sind:

Terpolymere aus Acrylsäure, Alkylacrylat und N-Alkylacrylamid (INCI-Bezeichnung: Acrylates/Acrylamide Copolymer), insbesondere Terpolymere aus Acrylsäure, Ethylacrylat und N-tert-Butylacrylamid; vernetzte oder unvernetzte Vinylacetat/Crotonsäure Copolymere (INCI-Bezeichnung: VA/Crotonates Copolymer); Copolymere aus ein oder mehreren C1-C5-Alkylacrylaten, insbesondere C2-C4-Alkylacrylaten und mindestens einem Monomer ausgewählt aus Acrylsäure oder Methacrylsäure (INCI-Bezeichnung: Acrylates Copolymer), z.B. Terpolymere aus tert.-Butylacrylat, Ethylacrylat und Methacrylsäure; Natriumpolystyrolsulfonat; Vinylacetat/Crotonsäure/ Vinylalkanoat Copolymere, z.B. Copolymere aus Vinylacetat, Crotonsäure und Vinylpropionat; Copolymere aus Vinylacetat, Crotonsäure und Vinylneodecanoat (INCI-Bezeichnungen: VA/Crotonates/Vinyl Propionate Copolymer, VA/Crotonates/Vinyl Neodecanoate Copolymer); Aminomethylpropanol-Acrylat Copolymere; Copolymere aus Vinylpyrrolidon und mindestens einem weiteren Monomer ausgewählt aus Acrylsäure und Methacrylsäure sowie ggf. Acrylsäureestern und Methacrylsäureestern; Copolymere aus Methylvinylether und Maleinsäuremonoalkylestern (INCI-Bezeichnungen: Ethylester of PVM/MA Copolymer, Butylester of PVM/MA Copolymer); Aminomethylpropanolsalze von Copolymeren aus Allylmethacrylat und mindestens einem weiteren Monomer ausgewählt aus Acrylsäure, und Methacrylsäure sowie ggf. Acrylsäureestern und Methacrylsäureestern; vernetzte Copolymere aus Ethylacrylat und Methacrylsäure; Copolymere aus Vinylacetat, Mono-n-butylmaleat und Isobornylacrylat; Copolymere aus zwei oder mehr Monomeren ausgewählt aus Acrylsäure und Methacrylsäure sowie ggf. Acrylsäureestern und Methacrylsäureestern; Copolymere aus Octylacrylamid und mindestens einem Monomeren ausgewählt aus Acrylsäure und Methacrylsäure sowie ggf. Acrylsäureestern und Methacrylsäureestern; Polyester aus Diglycol, Cyclohexandimethanol, Isophtalsäure und Sulfoisophtalsäure, wobei die Alkylgruppen der vorstehend genannten Polymere in der Regel vorzugsweise 1, 2, 3 oder 4 C-Atome aufweisen.

Zwitterionische Polymere weisen gleichzeitig mindestens eine anionische und mindestens eine kationische Ladung auf. Amphotere Polymere weisen mindestens ein saure Gruppe (z.B. Carbonsäure- oder Sulphonsäuregruppe) und mindestens eine basische Gruppe (z.B. Aminogruppe) auf. Säuregruppen können mittels üblicher Basen wie z.B. organischer Amine oder Alkali- oder Erdalkalihydroxide deprotoniert sein.

Bevorzugte zwitterionische oder amphotere Polymere sind: Copolymere gebildet aus Alkylacrylamid, Alkylaminoalkylmethacrylat und zwei oder mehr Monomeren aus Acrylsäure und Methacrylsäure sowie ggf. deren Estern, insbesondere Copolymere aus Octylacrylamid, Acrylsäure, Butylaminoethylmethacrylat, Methylmethacrylat und Hydroxypropylmethacrylat (INCI-Bezeichnung: Octylacrylamide/Acrylates/Butylaminoethyl Methacrylate Copolymer); Copolymere, welche gebildet sind aus mindestens einer ersten Monomerart, welche quaternäre Amingruppen aufweist und mindestens einer zweiten Monomerart, welche Säuregruppen aufweist; Copolymere aus Fettalkoholacrylaten, Alkylaminoxidmethacrylat und mindestens einem Monomeren ausgewählt aus Acrylsäure und Methacrylsäure sowie ggf. Acrylsäureestern und Methacrylsäureestern, insbesondere Copolymere aus Laurylacrylat, Stearylacrylat, Ethylaminoxidmethacrylat und mindestens einem Monomeren ausgewählt aus Acrylsäure und Methacrylsäure sowie ggf. deren Estern; Copolymere aus Methacryloylethylbetain und mindestens einem Monomeren ausgewählt aus Methacrylsäure und Methacrylsäureestern; Copolymere aus Acrylsäure, Methylacrylat und Methacrylamidopropyltrimethylammoniumchlorid (INCI-Bezeichnung: Polyquaternium-47); Copolymere aus Acrylamidopropyltrimethylammoniumchlorid und Acrylaten oder Copolymere aus Acrylamid, Acrylamidopropyltrimethylammoniumchlorid, 2-Amidopropylacrylamidsulfonat und Dimethylaminopropylamin (INCI-Bezeichnung: Polyquaternium-43); Oligomere oder Polymere, herstellbar aus quaternären Crotonbetainen oder quaternären Crotonbetainestern.

Kationische Polymere sind Polymere mit kationischen oder kationisierbaren Gruppen, insbesondere primären, sekundären, tertiären oder quaternären Amingruppen. Die kationische Ladungsdichte beträgt vorzugsweise 1 bis 7 meq/g. Die kationischen Polymere enthalten vorzugsweise quaternäre Amingruppen. Die kationischen Polymere können Homo- oder Copolymere sein, wobei die quaternären Stickstoffgruppen entweder in der Polymerkette oder vorzugsweise als Substituent an einem oder mehreren der Monomeren enthalten sind. Die Ammoniumgruppen enthaltenden Monomere können mit nicht kationischen Monomeren copolymerisiert sein. Geeignete kationische Monomere sind ungesättigte, radikalisch polymerisierbare Verbindungen, welche mindestens eine kationische Gruppe tragen, insbesondere ammoniumsubstituierte Vinylmonomere wie z.B. Trialkylmethacryloxyalkylammonium, Trialkylacryloxyalkylammonium, Dialkyldiallylammonium und quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, Alkylvinylpyridinium, oder Alkylvinylpyrrolidon Salze. Die Alkylgruppen dieser Monomere sind vorzugsweise niedere Alkylgruppen wie z.B. C1- bis C7-Alkylgruppen, besonders bevorzugt C1- bis C3-Alkylgruppen.

Die Ammoniumgruppen enthaltenden Monomere können mit nicht kationischen Monomeren copolymerisiert sein. Geeignete Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylcaprolactam, Vinylpyrrolidon, Vinylester, z.B. Vinylacetat, Vinylalkohol, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Geeignete Polymere mit quaternären Amingruppen sind z.B. die im CTFA Cosmetic Ingredient Dictionary unter den Bezeichnungen Polyquaternium beschriebenen Polymere wie Methylvinylimidazoliumchlorid/Vinylpyrrolidon Copolymer (Polyquaternium-16) oder quaternisiertes Vinylpyrrolidon/Dimethylaminoethylmethacrylat Copolymer (Polyquaternium-11) sowie quaternäre Silikonpolymere bzw. -oligomere wie z.B. Silikonpolymere mit quaternären Endgruppen (Quaternium-80).

### Bevorzugte kationische Polymere auf synthetischer Basis:

Poly(dimethyldiallylammoniumchlorid); Copolymere aus Acrylamid und Dimethyldiallylammoniumchlorid; quaternäre Ammoniumpolymere, gebildet durch die Reaktion von Diethylsulfat und einem Copolymer aus Vinylpyrrolidon und Dimethylaminoethylmethacrylat, insbesondere Vinylpyrrolidon/Dimethylaminoethylmethacrylatmethosulfat Copolymer (z.B. Gafquat® 755 N, Gafquat® 734); quaternäre Ammoniumpolymere aus Methylvinylimidazoliumchlorid und Vinylpyrrolidon (z.B. LUVIQUAT® HM 550); Polyquaternium-35; Polyquaternium-57; Polymer aus Trimethylammonium-ethyl-methacrylatchlorid; Terpolymere aus Dimethyldiallylammoniumchlorid, Natriumacrylat und Acrylamid (z.B. Merquat® Plus 3300); Copolymere aus Vinylpyrrolidon, Dimethylaminopropylmethacrylamid und Methacryloylaminopropyllauryldimethylammoniumchlorid; Terpolymere aus Vinylpyrrolidon, Dimethylaminoethylmethacrylat und Vinylcaprolactam (z.B. Gaffix® VC 713); Vinylpyrrolidon/Methacrylamidopropyltrimethylammoniumchlorid Copolymere (z.B. Gafquat® HS 100); Copolymere aus Vinylpyrrolidon und Dimethylaminoethylmethacrylat; Copolymere aus Vinylpyrrolidon, Vinylcaprolactam und Dimethylaminopropylacrylamid; Poly- oder Oligoester, aufgebaut aus mindestens einer ersten Monomerart, die ausgewählt ist aus mit mindestens einer quaternären Ammoniumgruppe substituierten Hydroxysäure; endständig mit quaternären Ammoniumgruppen substituierte Dimethylpolysiloxane.

Geeignete kationische Polymere, die von natürlichen Polymeren abgeleitet sind, sind insbesondere kationische Derivate von Polysacchariden, beispielsweise kationische Derivate von Cellulose, Stärke oder Guar. Geeignet sind weiterhin Chitosan und Chitosanderivate. Kationische Polysaccharide haben z.B. die allgemeine Formel

G-O-B-N⁺R^{a}R^{b}R^{c} X⁻

G ist ein Anhydroglucoserest, beispielsweise Stärke- oder Celluloseanhydroglucose;
B ist eine divalente Verbindungsgruppe, beispielsweise Alkylen, Oxyalkylen, Polyoxyalkylen oder Hydroxyalkylen;
R^{a}, R^{b} und R^{c} sind unabhängig voneinander Alkyl, Aryl, Alkylaryl, Arylalkyl, Alkoxyalkyl oder Alkoxyaryl mit jeweils bis zu 18 C-Atomen, wobei die Gesamtzahl der C-Atome in R^{a}, R^{b} und R^{c} vorzugsweise maximal 20 ist;
X ist ein übliches Gegenanion, beispielsweise ein Halogen, Acetat, Phosphat, Nitrat oder Alkylsulfat, vorzugsweise ein Chlorid. Kationische Cellulosen sind z.B. solche mit den INCI-Bezeichnungen Polyquaternium-10 oder Polyquaternium-24. Ein geeignetes kationisches Guarderivat hat z.B. die INCI-Bezeichnung Guar Hydroxypropyltrimonium Chloride.

Besonders bevorzugte kationaktive Stoffe sind Chitosan, Chitosansalze und Chitosan-Derivate. Bei den erfindungsgemäß einzusetzenden Chitosanen handelt es sich um vollständig oder partiell deacetylierte Chitine. Das Molekulargewicht kann über ein breites Spektrum verteilt sein, beispielsweise von 20.000 bis ca. 5 Millionen g/mol, z.B. von 30.000 bis 70.000 g/mol. Vorzugsweise liegt das Molekulargewicht jedoch über 100.000 g/mol, besonders bevorzugt von 200.000 bis 700.000 g/mol. Der Deacetylierungsgrad beträgt vorzugsweise 10 bis 99%, besonders bevorzugt 60 bis 99%. Ein bevorzugtes Chitosansalz ist Chitosoniumpyrrolidoncarboxylat, z.B. Kytamer® PC mit einem Molekulargewicht von ca. 200.000 bis 300.000 g/mol und Deacetylierung von 70 bis 85%. Als Chitosanderivate kommen quaternierte, alkylierte oder hydroxyalkylierte Derivate, z.B. Hydroxyethyl-, Hydroxypropyl- oder Hydroxybutylchitosan in Betracht. Die Chitosane oder Chitosanderivate liegen vorzugsweise in neutralisierter oder partiell neutralisierter Form vor. Der Neutralisationsgrad liegt vorzugsweise bei mindestens 50%, besonders bevorzugt zwischen 70 und 100%, bezogen auf die Anzahl der freien Basengruppen. Als Neutralisationsmittel können prinzipiell alle kosmetisch verträglichen anorganischen oder organischen Säuren verwendet werden wie beispielsweise Ameisensäure, Weinsäure, Äpfelsäure, Milchsäure, Zitronensäure, Pyrrolidoncarbonsäure, Salzsäure u.a., von denen die Salzsäure und Ameisensäure besonders bevorzugt sind.

Bevorzugte kationische Polymere auf natürlicher Basis:
kationische Cellulosederivate aus Hydroxyethylcellulose und Diallyldimethylammoniumchlorid; kationische Cellulosederivate aus Hydroxyethylcellulose und mit Trimethylammonium substituiertem Epoxid; Chitosan und dessen Salze; Hydroxyalkylchitosane und deren Salze; Alkyl-hydroxyalkylchitosane und deren Salze; N-Hydroxyalkylchitosanalkylether.

Nichtionische haarfestigende Polymere können synthetischen oder natürlichen Ursprungs sein. Unter natürlichen Polymeren werden auch chemisch modifizierte Polymere natürlichen Ursprungs verstanden.
Geeignete synthetische, nichtionische filmbildende, haarfestigende Polymere sind Homo- oder Copolymere, die aus mindestens einem der folgenden Monomere aufgebaut sind:
Vinylpyrrolidon, Vinylcaprolactam, Vinylester wie z.B. Vinylacetat, Vinylalkohol, Acrylamid, Methacrylamid, Alkyl- und Dialkylacrylamid, Alkyl- und Dialkylmethacrylamid, Alkylacrylat, Alkylmethacrylat, Propylenglykol oder Ethylenglykol, wobei die Alkylgruppen dieser Monomere vorzugsweise C1- bis C7-Alkylgruppen, besonders bevorzugt C1- bis C3-Alkylgruppen sind. Geeignet sind z.B. Homopolymere des Vinylcaprolactams, des Vinylpyrrolidons oder des N-Vinylformamids. Weitere geeignete synthetische filmbildende, nicht-ionische, haarfestigende Polymere sind z.B. Copolymerisate aus Vinylpyrrolidon und Vinylacetat, Terpolymere aus Vinylpyrrolidon, Vinylacetat und Vinylpropionat, Polyacrylamide; Polyvinylalkohole; Terpolymere aus Vinylpyrrolidon, Methacrylamid und Vinylimidazol sowie Polyethylenglykol/Polypropylenglykol Copolymere. Geeignete natürliche filmbildende Polymere sind z.B. Cellulosederivate, z.B. Hydroxyalkylcellulose.

Bevorzugte nichtionische Polymere sind:
Polyvinylpyrrolidon, Polyvinylcaprolactam, Vinylpyrrolidon/Vinylacetat Copolymere, Polyvinylalkohol, Isobutylen/Ethylmaleimid/Hydroxyethylmaleimid Copolymer; Copolymere aus Vinylpyrrolidon, Vinylacetat und Vinylpropionat.

In einer Ausführungsform enthält das erfindungsmäße Mittel als haarpflegenden Zusatzstoff mindestens eine Silikonverbindung in einer Menge von vorzugsweise 0,01 bis 15 Gew.%, besonders bevorzugt von 0,1 bis 5 Gew.%, vorzugsweise in klar, z.B. als Mikroemulsion einemulgierter Form. Die Silikonverbindungen umfassen flüchtige und nicht-flüchtige Silikone und in dem Mittel lösliche und unlösliche Silikone. Bei einer Ausführungsform handelt es sich um hochmolekulare Silikone mit einer Viskosität von 1.000 bis 2.000.000 cSt bei 25°C, vorzugsweise 10.000 bis 1.800.000 oder 100.000 bis 1.500.000. Die Silikonverbindungen umfassen Polyalkyl- und Polyarylsiloxane, insbesondere mit Methyl-, Ethyl-, Propyl-, Phenyl-, Methylphenyl- und Phenylmethylgruppen. Bevorzugt sind Polydimethylsiloxane, Polydiethylsiloxane, Polymethylphenylsiloxane. Bevorzugt sind auch glanzgebende, arylierte Silikone mit einem Brechungsindex von mindestens 1,46, oder mindestens 1,52. Die Silikonverbindungen umfassen insbesondere die Stoffe mit den INCI-Bezeichnungen Cyclomethicone, Dimethicone, Dimethiconol, Dimethicone Copolyol, Phenyl Trimethicone, Amodimethicone, Trimethylsilylamodimethicone, Stearyl Siloxysilicate, Polymethylsilsesquioxane, Dimethicone Crosspolymer. Geeignet sind auch Silikonharze und Silikonelastomere, wobei es sich um hochvernetzte Siloxane handelt.
Bevorzugte Silikone sind:
cyclische Dimethylsiloxane, lineare Polydimethylsiloxane, Blockpolymere aus Polydimethylsiloxan und Polyethylenoxid und/oder Polypropylenoxid, Polydimethylsiloxane mit end- oder seitenständigen Polyethylenoxid- oder Polypropylenoxidresten, Polydimethylsiloxane mit endständigen Hydroxylgruppen, phenylsubstituierte Polydimethylsiloxane, Silikonemulsionen, Silkonelastomere, Silikonwachse, Silikongums und aminosubstituierte Silikone.

In einer Ausführungsform enthält das erfindungsmäße Mittel 0,01 bis 20, besonders bevorzugt von 0,05 bis 10, ganz besonders bevorzugt von 0,1 bis 5 Gew.% an mindestens einem haarkonditionierenden Zusatzstoff, ausgewählt aus Betain; Panthenol; Panthenylethylether; Sorbitol; Proteinhydrolysaten; Pflanzenextrakten.

In einer Ausführungsform enthält das erfindungsmäße Mittel 0,01 bis 20, besonders bevorzugt von 0,05 bis 10, ganz besonders bevorzugt von 0,1 bis 5 Gew.% an mindestens einem Tensid. Das Tensid kann nichtionisch, anionisch, kationisch oder zwitterionisch sein und kann insbesondere zur Einemulgierung oder Lösungsvermittlung von hydrophoben Zusatzstoffen wie z.B. Parfümölen dienen. Bevorzugte Gele enthalten mindestens ein Parfümöl in optisch klar einemulgierter Form.

Nichtionische Tenside sind z.B.
- Ethoxylierte Fettalkohole, Fettsäuren, Fettsäureglyceride oder Alkylphenole, insbesondere Anlagerungsprodukte von 2 bis 30 mol Ethylenoxid und/oder 1 bis 5 mol Propylenoxid an C8- bis C22-Fettalkohole, an C12- bis C22-Fettsäuren oder an Alkylphenole mit 8 bis 15 C-Atomen in der Alkylgruppe
- C12- bis C22-Fettsäuremono- und -diester von Anlagerungsprodukten von 1 bis 30 mol Ethylenoxid an Glycerin
- Anlagerungsprodukte von 5 bis 60 mol Ethylenoxid an Rizinusöl oder an gehärtetes (hydriertes) Rizinusöl.
- Fettsäurezuckerester, insbesondere Ester aus Saccharose und ein oder zwei C8- bis C22-Fettsäuren, INCI: Sucrose Cocoate, Sucrose Dilaurate, Sucrose Distearate, Sucrose Laurate, Sucrose Myristate, Sucrose Oleate, Sucrose Palmitate, Sucrose Ricinoleate, Sucrose Stearate
- Ester aus Sorbitan und ein, zwei oder drei C8- bis C22-Fettsäuren und einem Ethoxylierungsgrad von 4 bis 20
- Polyglycerylfettsäureester, insbesondere aus ein, zwei oder mehreren C8- bis C22-Fettsäuren und Polyglycerin mit vorzugsweise 2 bis 20 Glyceryleinheiten
- Alkylglucoside, Alkyloligoglucoside und Alkylpolyglucoside mit C8- bis C22-Alkylgruppen, z.B. Decyl Glucoside oder Lauryl Glucoside,

Anionische Tenside sind z.B. Salze und Ester von Carbonsäuren, Alkylethersulfate und Alkylsulfate, Fettalkoholethersulfate, Sulfonsäure und ihre Salze (z.B. Sulfosuccinate oder Fettsäureisethienate), Phosphorsäureester und ihre Salze, Acylaminosäuren und ihre Salze. Eine ausführliche Beschreibung dieser anionischen Tenside ist der Publikation "FIEDLER - Lexikon der Hilfsstoffe", Band 1, fünfte Auflage (2002), Seiten 97 bis 102, zu entnehmen, auf die hiermit ausdrücklich Bezug genommen wird. Bevorzugte Tenside sind Mono-, Di- und/oder Triester der Phosphorsäure mit Anlagerungsprodukten von 2 bis 30 mol Ethylenoxid an C8- bis C22-Fettalkohole.

Amphotere Tenside sind z.B. Derivate aliphatischer quaternärer Ammonium-, Phosphonium- und Sulfoniumverbindungen der Formel 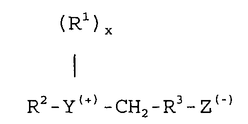
wobei R1 eine geradkettige oder verzweigtkettige Alkyl-, Alkenyl- oder Hydroxyalkylgruppe mit 8 bis 18 C-Atomen und 0 bis etwa 10 Ethylenoxideinheiten und 0 bis 1 Glycerineinheit darstellt; Y eine N-, P- oder S-haltige Gruppe ist; R2 eine Alkyl- oder Monohydroxyalkylgruppe mit 1 bis 3 C-Atomen ist; X gleich 1 ist, falls Y ein Schwefelatom ist und X gleich 2 ist, wenn Y ein Stickstoffatom oder ein Phosphoratom ist; R3 eine Alkylen- oder Hydroxyalkylengruppe mit 1 bis 4 C-Atomen ist und Z⁽⁻⁾ eine Carboxylat-, Sulfat-, Phosphonat- oder Phosphatgruppe darstellt.
Andere amphotere Tenside wie Betaine sind ebenso geeignet. Beispiele für Betaine umfassen C8- bis C18-Alkylbetaine wie Cocodimethylcarboxymethylbetain, Lauryldimethylcarboxymethylbetain, Lauryldimethylalphacarboxyethylbetain, Cetyldimethylcarboxymethylbetain, Oleyldimethylgammacarboxypropylbetain und Lauryl-bis(2-hydroxypropyl)alphacarboxyethylbetain; C8- bis C18-Sulfobetaine wie Cocodimethylsulfopropylbetain, Stearyldimethylsulfopropylbetain, Lauryldimethylsulfoethylbetain, Laurylbis-(2-hydroxyethyl)sulfopropylbetain; die Carboxylderivate des Imidazols, die C8- bis C18-Alkyldimethylammoniumacetate, die C8- bis C18-Alkyldimethylcarbonylmethylammoniumsalze sowie die C8- bis C18-Fettsäurealkylamidobetaine wie beispielsweise Kokosfettsäureamidopropylbetain und N-Kokosfettsäureamidoethyl-N-[2-(carboxymethoxy)-ethyl]-glycerin (CTFA-Name: Cocoamphocarboxyglycinate).

Kationische Tenside enthalten Aminogruppen oder quaternisierte hydrophile Ammoniumgruppen, welche in Lösung eine positive Ladung tragen und durch die allgemeine Formel

N⁽⁺⁾R¹R²R³R⁴ X⁽⁻⁾

dargestellt werden können, wobei R1 bis R4 unabhängig voneinander aliphatische Gruppen, aromatische Gruppen, Alkoxygruppen, Polyoxyalkylengruppen, Alkylamidogruppen, Hydroxyalkylgruppen, Arylgruppen oder Alkarylgruppen mit 1 bis 22 C-Atomen bedeuten, wobei mindestens ein Rest mindestens 6, vorzugsweise mindestens 8 C-Atome aufweist und X- ein Anion darstellt, beispielsweise ein Halogen, Acetat, Phosphat, Nitrat oder Alkylsulfat, vorzugsweise ein Chlorid. Die aliphatischen Gruppen können zusätzlich zu den Kohlenstoffatomen und den Wasserstoffatomen auch Querverbindungen oder andere Gruppen wie beispielsweise weitere Aminogruppen enthalten.
Beispiele für geeignete kationische Tenside sind die Chloride oder Bromide von Alkyldimethylbenzylammoniumsalzen, Alkyltrimethylammoniumsalze, beispielsweise Cetyltrimethylammoniumchlorid oder -bromid, Tetradecyltrimethylammoniumchlorid oder -bromid, Alkyldimethylhydroxyethylammoniumchloride oder -bromide, die Dialkyldimethylammoniumchloride oder -bromide, Alkylpyridiniumsalze, beispielsweise Lauryl- oder Cetylpyridiniumchlorid, Alkylamidoethyltrimethylammoniumethersulfate sowie Verbindungen mit kationischem Charakter wie Aminoxide, beispielsweise Alkylmethylaminoxide oder Alkylaminoethyldimethylaminoxide. Besonders bevorzugt sind C8-22-Alkyldimethylbenzylammoniumverbindungen, C8-22- Alkyltrimethylammoniumverbindungen, insbesondere Cetyltrimethylammoniumchlorid, C8-22-Alkyldimethylhydroxyethylammoniumverbindungen, Di-(C8-22-alkyl)-dimethylammoniumverbindungen, C8-22-Alkylpyridiniumsalze, C8-22- Alkylamidoethyltrimethylammoniumethersulfate, C8-22- Alkylmethylaminoxide, C8-22-Alkylaminoethyldimethylaminoxide.

In einer Ausführungsform enthält das erfindungsgemäße Mittel 0,01 bis 5, besonders bevorzugt von 0,05 bis 1 Gew.% an mindestens einem Konservierungsmittel. Geeignete Konservierungsmittel sind die im International Cosmetic Ingredient Dictionary and Handbook, 9. Auflage mit der Funktion "Preservatives" aufgeführten Stoffe, z.B. Phenoxyethanol, Benzylparaben, Butylparaben, Ethylparaben, Isobutylparaben, Isopropylparaben, Methylparaben, Propylparaben, Iodopropinylbutylcarbamat, Methyldibromoglutaronitril, DMDM Hydantoin.

In einer weiteren Ausführungsform enthält das erfindungsgemäße Mittel mindestens ein Pigment. Hierbei kann es sich um farbige Pigmente handeln, welche der Produktmasse oder dem Haar Farbeffekte verleihen oder es kann sich um Glanzeffektpigmente handeln, welche der Produktmasse oder dem Haar Glanzeffekte verleihen. Die Farb- oder Glanzeffekte am Haar sind vorzugsweise temporär, d.h. sie halten bis zur nächsten Haarwäsche und können durch Waschen der Haare mit üblichen Shampoos wieder entfernt werden. Die Pigmente liegen in der Produktmasse in ungelöster Form vor und können in einer Menge von 0,01 bis 25 Gew.%, besonders bevorzugt von 5 bis 15 Gew.% enthalten sein. Die bevorzugte Teilchengröße beträgt 1 bis 200 µm, insbesondere 3 bis 150 µm, besonders bevorzugt 10 bis 100 µm. Die Pigmente sind im Anwendungsmedium praktisch unlösliche Farbmittel und können anorganisch oder organisch sein. Auch anorganisch-organische Mischpigmente sind möglich. Bevorzugt sind anorganische Pigmente. Der Vorteil der anorganischen Pigmente ist deren ausgezeichnete Licht-, Wetter- und Temperaturbeständigkeit. Die anorganischen Pigmente können natürlichen Ursprungs sein, beispielsweise hergestellt aus Kreide, Ocker, Umbra, Grünerde, gebranntem Terra di Siena oder Graphit. Bei den Pigmenten kann es sich um Weißpigmente wie z.B. Titandioxid oder Zinkoxid, um Schwarzpigmente wie z.B. Eisenoxidschwarz, Buntpigmente wie z.B. Ultramarin oder Eisenoxidrot, um Glanzpigmente, Metalleffekt-Pigmente, Perlglanzpigmente sowie um Fluoreszenz- oder Phosphoreszenzpigmente handeln, wobei vorzugsweise mindestens ein Pigment ein farbiges, nicht-weißes Pigment ist. Geeignet sind Metalloxide, -hydroxide und -oxidhydrate, Mischphasenpigmente, schwefelhaltige Silicate, Metallsulfide, komplexe Metallcyanide, Metallsulfate, -chromate und -molybdate sowie die Metalle selbst (Bronzepigmente). Geeignet sind insbesondere Titandioxid (CI 77891), schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510), Carmine (Cochineal).

Besonders bevorzugt sind Perlglanz- und Farbpigmente auf Mica- bzw. Glimmerbasis welche mit einem Metalloxid oder einem Metalloxychlorid wie Titandioxid oder Wismutoxychlorid sowie gegebenenfalls weiteren farbgebenden Stoffen wie Eisenoxiden, Eisenblau, Ultramarine, Carmine etc. beschichtet sind und wobei die Farbe durch Variation der Schichtdicke bestimmt sein kann. Derartige Pigmente werden beispielsweise unter den Handelsbezeichnungen Rona®, Colorona®, Dichrona® und Timiron® von der Firma Merck, Deutschland vertrieben.

Organische Pigmente sind beispielsweise die natürlichen Pigmente Sepia, Gummigutt, Knochenkohle, Kasseler Braun, Indigo, Chlorophyll und andere Pflanzenpigmente. Synthetische organische Pigmente sind beispielsweise Azo-Pigmente, Anthrachinoide, Indigoide, Dioxazin-, Chinacridon-, Phtalocyanin-, Isoindolinon-, Perylen- und Perinon-, Metallkomplex-, Alkaliblau- und Diketopyrrolopyrrol-Pigmente.

In einer Ausführungsform enthält das erfindungsmäße Mittel 0,01 bis 10, besonders bevorzugt von 0,05 bis 5 Gew.% an mindestens einem partikelförmigen Stoff. Geeignete Stoffe sind z.B. Stoffe, die bei Raumtemperatur (25°C) fest sind und in Form von Partikeln vorliegen. Geeignet sind etwa Silica, Silikate, Aluminate, Tonerden, Mica, Salze, insbesondere anorganische Metallsalze, Metalloxide, z.B. Titandioxid, Minerale und Polymerpartikel.

Die Partikel liegen in dem Mittel ungelöster, vorzugsweise stabil dispergierter Form vor und können sich nach Aufbringen auf das Haar und Verdampfen des Lösungsmittels auf dem Haar in fester Form abscheiden. Eine stabile Dispergierung kann erreicht werden, indem die Zusammensetzung mit einer Fließgrenze versehen wird, die groß genug ist, um ein Absinken der Feststoffpartikel zu verhindern. Eine ausreichende Fließgrenze kann durch Verwendung von geeigneten Gelbildnern in geeigneter Menge eingestellt werden.

Bevorzugte partikelförmige Stoffe sind Silica (Kieselgel, Siliciumdioxid) und Metallsalze, insbesondere anorganische Metallsalze, wobei Silica besonders bevorzugt ist. Metallsalze sind z.B. Alkali- oder Erdalkalihalogenide wie Natriumchlorid oder Kaliumchlorid; Alkali- oder Erdalkalisulfate wie Natriumsulfat oder Magnesiumsulfat.

Als Lösungsmittel wird in dem erfindungsgemäßen Gel bevorzugt Wasser oder ein Wasser/Alkohol-Gemisch eingesetzt. Der Alkoholgehalt kann bis zu 50 Gew.% betragen. Als Alkohole können insbesondere die für kosmetische Zwecke üblicherweise verwendeten niederen Alkohole mit 1 bis 4 C-Atomen wie z.B. Ethanol und Isopropanol enthalten sein. Das erfindungsgemäße Mittel kann in einem pH-Bereich von 2,0 bis 9,5 vorliegen. Besonders bevorzugt ist der pH-Bereich zwischen 4 und 8. Als zusätzliche Co-Solventien können organische Lösungsmittel oder ein Gemisch aus Lösungsmitteln mit einem Siedepunkt unter 400°C in einer Menge von 0,1 bis 15 Gew.% bevorzugt von 1 bis 10 Gew.% enthalten sein. Besonders geeignet als zusätzliche Co-Solventien sind Polyole wie z.B. Glycerin, Ethylenglykol und Propylenglykol.

Das erfindungsgemäße Mittel kann in verschiedenen Applikationsformen Anwendung finden, wie beispielsweise als Gel zur Ausbringung aus einer Tube, als Flüssiggel, als versprühbares Gel mit einer geeigneten Sprühvorrichtung oder als Schaumgel mit einer geeigneten Vorrichtung zum Verschäumen.

Die Viskosität beträgt vorzugsweise mindestens 250 mPa s, insbesondere von 500 bis 50.000 mPa s, besonders bevorzugt von 1.000 bis 15.000 mPa s oder von 5.000 bis 15.000 mPa s, gemessen mit einem Haake Viskotester 550, Messkörper SV (DIN 53019) bei 25°C und Schergeschwindigkeit von 12,9 s⁻¹.

Eine bevorzugte Ausführungsform ist ein Haarstylinggel mit einem Gehalt an
(A) 0,5 bis 10 Gew.% mindestens eines Gelbildner, ausgewählt aus Acrylat/C10-30 Alkylacrylat Crosspolymeren, Carbomeren, Xanthangum und Acrylat/Ceteth-20 Itaconat Copolymer;
(B) 3 bis 15 Gew.% mindestens eines UV-Filters, ausgewählt aus organischen Verbindungen mit mindestens einer Benzimidazoleinheit und mindestens einer Sulfonsäuregruppe und deren Salzen und
(C) 0,1 bis 15 Gew.% mindestens eines haarfestigenden Polymers ausgewählt aus anionischen und nichtionischen Polymeren.

Die nachfolgenden Beispiele sollen den Gegenstand der Erfindung näher erläutern. Die angegebenen Polymergehalte beziehen sich, wenn nicht anders angegeben, jeweils auf den Feststoffgehalt.

### Beispiele

Es werden die folgenden Produkte eingesetzt:
Heliopan® Hydro: 2-Phenyl-1H-benzimidazol-5-sulfonsäure
Heliopan® AP: 2,2'-(1,4-Phenylen)bis-(1H-benzimidazol-4,6disulfonsäure)
Ultrez® 21: Acrylat/C10-30 Alkylacrylat Crosspolymer Aquaflex® SF 40: Vinylpyrrolidon/Vinylcaprolactam/Dimethylaminopropylacrylamid Copolymer
Luviskol® VA 64: VP/VA Copolymer
Structure® 3001: Acrylates/Ceteth-20 Itaconate Copolymer
Aculyn® 48: PEG-150/Stearylalkohol/SMDI Copolymer, 19%ig in Wasser

**Beispiel 1: Haarstylinggel - normaler Halt**

| | |
|---|---|
| Ultrez® 21 | 2,0 |
| Polyvinylpyrrolidon K 90 | 1,8 |
| Heliopan® Hydro | 2,0 |
| Heliopan® AP | 2,0 |
| Aminomethylpropanol 95% | 3,0 |
| PEG-40 Hydrogenated Castor Oil | 0,2 |
| Parfüm | 0,2 |
| Wasser | Ad 100 |

**Beispiel 2: Haarstylinggel - starker Halt**

| | |
|---|---|
| Carbomer (Carbopol®) | 2,0 |
| Vinylacetat/Crotonat Copolymer (Luviset® CA 66) | 2,5 |
| Heliopan® Hydro | 8,0 |
| Aminomethylpropanol 95% | 4,0 |
| PEG-40 Hydrogenated Castor Oil | 0,2 |
| Panthenol | 0,1 |
| Parfüm | 0,2 |
| Wasser | Ad 100 |

**Beispiel 3: Haarstylinggel - starker Halt**

| | |
|---|---|
| Xanthangum (Amaze® XT) | 1,5 |
| Aquaflex® SF 40 | 2,7 |
| Heliopan® Hydro | 6,0 |
| Heliopan® AP | 6,0 |
| Aminomethylpropanol 95% | 3,6 |
| PEG-25 PABA | 0,5 |
| PEG-40 Hydrogenated Castor Oil | 0,2 |
| Panthenol | 0,1 |
| Parfüm | 0,2 |
| Wasser | Ad 100 |

**Beispiel 4: Haarstylinggel - normaler Halt**

| | |
|---|---|
| Ultrez® 21 | 2,0 |
| Glycerin | 5,2 |
| Propylene Glycol | 4,0 |
| Heliopan® AP | 6,0 |
| Aminomethylpropanol 95% | 3,6 |
| Vinylpyrrolidon/Vinylacetat Copolymer | 3,0 |
| Wasser | Ad 100 |

**Beispiel 5: Haarstylinggel-Spray**

| | |
|---|---|
| Xanthangum (Amaze® XT) | 1,0 |
| Vinylpyrrolidon/Vinylacetat Copolymer | 1,5 |
| Heliopan® Hydro | 2,0 |
| Heliopan® AP | 2,0 |
| Aminomethylpropanol 95% | 1,2 |
| Parfüm | 0,15 |
| Ethanol | 10,0 |
| Wasser | Ad 100 |

**Beispiel 6: Haarstyling-Sprühgel**

| | |
|---|---|
| Ammonium Acryloyoldimethyltaurat/Vinylpyrrolidon Copolymer (Aristoflex® AVC) | 1,0 |
| Vinylpyrrolidon/Vinylacetat Copolymer | 3,0 |
| Ethanol | 18,0 |
| Heliopan® Hydro | 8,0 |
| Aminomethylpropanol 95% | 2,7 |
| PEG-40 | 0,2 |
| Parfüm | 0,2 |
| Wasser | Ad 70 |

**Beispiel 7: Haarstyling-Sprühgel**

| | |
|---|---|
| Structure® 3001 | 3,5 |
| Vinylpyrrolidon/Vinylacetat Copolymer | 3,0 |
| Ethanol | 8,0 |
| Heliopan® Hydro | 2,0 |
| Heliopan® AP | 2,0 |
| Aminomethylpropanol 95% | 2,8 |
| PEG-40 | 0,2 |
| Parfüm | 0,2 |
| Aculyn® 48 | 0,5 |
| Wasser | Ad 70 |

**Beispiel 8: Haarstylinggel - flüssig**

| | |
|---|---|
| Carbomer (Carbopol®) | 1,0 |
| Vinylacetat/Crotonat Copolymer (Luviset® CA 66) | 1,5 |
| Propylenglycol | 3,8 |
| Hydroxypropylguar | 0,3 |
| Heliopan® Hydro | 6,0 |
| Heliopan® AP | 6,0 |
| Aminomethylpropanol 95% | 3,8 |
| PEG-40 Hydrogenated Castor Oil | 0,18 |
| PPG-1-PEG-9 lauryl Glycol Ether | 0,18 |
| Parfüm | 0,15 |
| Ethanol | 16,5 |
| Wasser | Ad 100 |

**Beispiel 9: Haarstylinggel - flüssig**

| | |
|---|---|
| Carbomer (Carbopol®) | 1,5 |
| PEG-12 Dimethicone (Dow Corning Surfactant 193) | 1,5 |
| Structure® 3001 | 2,0 |
| Heliopan® AP | 6,0 |
| Aminomethylpropanol 95% | 3,7 |
| PPG-1-PEG-9 Laurylglykolether | 0,2 |
| Parfüm | 0,15 |
| Hydroxyethylcellulose | 0,4 |
| Ethanol | 16,5 |
| Wasser | Ad 100 |

**Beispiel 10: Haarstylinggel**

| | |
|---|---|
| Carbomer (Carbopol®) | 2,0 |
| Luviset® CA 66 | 2,5 |
| Heliopan® AP | 1,0 |
| Aminomethylpropanol 95% | 2,0 |
| PEG-40 Hydrogenated Castor Oil | 0,2 |
| Panthenol | 0,1 |
| Parfüm | 0,2 |
| Wasser | Ad 100 |

## Patentansprüche

1. Haarstylinggel mit einem Gehalt an einer Kombination von
(A) mindestens einem Gelbildner;
(B) mehr als 2 Gew.% mindestens eines UV-Filters und
(C) mindestens einem haarfestigenden Polymer.

2. Gel nach Anspruch 1, **dadurch gekennzeichnet, dass** das Gel einen Hautlichtschutzfaktor von mindestens 6 aufweist.

3. Gel nach einem der vorhergehenden Ansprüche, **dadurch gekennzeichnet, dass** der Gelbildner (A) in einer Menge von 0,5 bis 20 Gew.%, das haarfestigende Polymer (C) in einer Menge von 0,1 bis 15 Gew.% und/oder der UV-Filter (B) in einer Menge von 3 bis 15 Gew.% vorliegen.

4. Gel nach einem der vorhergehenden Ansprüche, **dadurch gekennzeichnet, dass** die Viskosität mindestens 250 mPa s bei 25°C und einer Schergeschwindigkeit von 12,9 s⁻¹ beträgt.

5. Gel nach einem der vorhergehenden Ansprüche, **dadurch gekennzeichnet, dass** der Gelbildner (A) ein Polymer ist, ausgewählt aus
- Copolymeren aus mindestens einer ersten Monomerart, die ausgewählt ist aus Acrylsäure und Methacrylsäure und mindestens einer zweiten Monomerart, die ausgewählt ist aus Estern der Acrylsäure und ethoxyliertem Fettalkohol;
- vernetzten Polyacrylsäuren;
- vernetzten Copolymeren aus mindestens einer ersten Monomerart, die ausgewählt ist aus Acrylsäure und Methacrylsäure und mindestens einer zweiten Monomerart, die ausgewählt ist aus Estern der Acrylsäure mit C10- bis C30-Alkoholen;
- Copolymeren aus mindestens einer ersten Monomerart, die ausgewählt ist aus Acrylsäure und Methacrylsäure und mindestens einer zweiten Monomerart, die ausgewählt ist aus Estern der Itaconsäure und ethoxyliertem Fettalkohol
- Copolymeren aus mindestens einer ersten Monomerart, die ausgewählt ist aus Acrylsäure und Methacrylsäure, mindestens einer zweiten Monomerart, die ausgewählt ist aus Estern der Itaconsäure und ethoxyliertem C10- bis C30-Alkohol und einer dritten Monomerart, ausgewählt aus C1- bis C4-Aminoalkylacrylaten;
- Copolymeren aus zwei oder mehr Monomeren, ausgewählt aus Acrylsäure, Methacrylsäure, Acrylsäureestern und Methacrylsäureestern;
- Copolymeren aus Vinylpyrrolidon und Ammoniumacryloyldimethyltaurat;
- Copolymeren aus Ammoniumacryloyldimethyltaurat und Monomeren ausgewählt aus Estern von Methacrylsäure und ethoxylierten Fettalkoholen;
- Glycerylpolyacrylat;
- Glycerylpolymethacrylat;
- Copolymeren aus mindestens einem C2-, C3- oder C4-Alkylen und Styrol;
- Polyurethanen;
- Copolymeren aus Polyethylenoxid, Fettalkoholen und gesättigtem Methylendiphenyldiisocyanat.
- Hydroxypropylstärkephosphat;
- Polyacrylamid;
- mit Decadien vernetztes Copolymer aus Maleinsäureanhydrid und Methylvinylether;
- Johannesbrotkernmehl; Guar-Gummi; Xanthan; Dehydroxanthan; Carrageenan; Karaya-Gummi; hydrolysierte Maisstärke; Hydroxyethylcellulose; Hydroxypropylcellulose; Hydroxypropylguar.

6. Gel nach einem der vorhergehenden Ansprüche, **dadurch gekennzeichnet, dass** der UV-Filter (B) mindestens eine Benzimidazol- oder Benzoxazol-Struktureinheit enthält und mindestens eine Sulfonsäuregruppe aufweist.

7. Gel nach einem der vorhergehenden Ansprüche, **dadurch gekennzeichnet, dass** als UV-Filter (B) eine Kombination von mindestens einem UV-A-Filter mit mindestens einem UV-B-Filter enthalten ist.

8. Gel nach einem der vorhergehenden Ansprüche, **dadurch gekennzeichnet, dass** der UV-Filter (B) ausgewählt ist aus Phenylbenzimidazolsulfonsäure und deren Salzen, Phenyldibenzimidazoltetrasulfonsäure und deren Salzen und deren Gemischen.

9. Gel nach einem der vorhergehenden Ansprüche, **dadurch gekennzeichnet, dass** das haarfestigende Polymer ausgewählt ist aus
- Polymeren mit anionischen oder anionisierbaren Gruppen, ausgewählt aus Terpolymeren aus Acrylsäure, Ethylacrylat und N-tert-Butylacrylamid; vernetzten oder unvernetzten Vinylacetat/Crotonsäure Copolymeren; Terpolymeren aus tert.-Butylacrylat, Ethylacrylat und Methacrylsäure; Natriumpolystyrolsulfonat; Copolymeren aus Vinylacetat, Crotonsäure und Vinylpropionat; Copolymeren aus Vinylacetat, Crotonsäure und Vinylneodecanoat; Aminomethylpropanol-Acrylat Copolymeren; Copolymeren aus Vinylpyrrolidon und mindestens einem weiteren Monomer ausgewählt aus Acrylsäure, Methacrylsäure, Acrylsäureestern und Methacrylsäureestern; Copolymeren aus Methylvinylether und Maleinsäuremonoalkylestern; Aminomethylpropanolsalze von Copolymeren aus Allylmethacrylat und mindestens einem weiteren Monomer ausgewählt aus Acrylsäure, Methacrylsäure, Acrylsäureestern und Methacrylsäureestern; vernetzten Copolymeren aus Ethylacrylat und Methacrylsäure; Copolymeren aus Vinylacetat, Mono-n-butylmaleat und Isobornylacrylat; Copolymeren aus zwei oder mehr Monomeren ausgewählt aus Acrylsäure, Methacrylsäure, Acrylsäureestern und Methacrylsäureestern, Copolymeren aus Octylacrylamid und mindestens einem Monomeren ausgewählt aus Acrylsäure, Methacrylsäure, Acrylsäureestern und Methacrylsäureestern; Polyestern aus Diglycol, Cyclohexandimethanol, Isophtalsäure und Sulfoisophtalsäure;
- Polymeren mit kationischen oder kationisierbaren Gruppen, ausgewählt aus kationischen Cellulosederivaten aus Hydroxyethylcellulose und Diallyldimethylammöniumchlorid; kationischen Cellulosederivaten aus Hydroxyethylcellulose und mit Trimethylammonium substituiertem Epoxid; Poly(dimethyldiallylammoniumchlorid); Copolymeren aus Acrylamid und Dimethyldiallylammoniumchlorid; quaternären Ammoniumpolymeren, gebildet durch die Reaktion von Diethylsulfat und einem Copolymer aus Vinylpyrrolidon und Dimethylaminoethylmethacrylat; quaternären Ammoniumpolymeren aus Methylvinylimidazoliumchlorid und Vinylpyrrolidon; Polyquaternium-35; Polymer aus Trimethylammonium-ethyl-methacrylatchlorid; Polyquaternium-57; endständig mit quaternären Ammoniumgruppen substituierte Dimethylpolysiloxane; Copolymer aus Vinylpyrrolidon, Dimethylaminopropylmethacrylamid und Methacryloylaminopropyllauryldimethylammoniumchlorid; Chitosan und dessen Salze; Hydroxyalkylchitosane und deren Salze; Alkyl-hydroxyalkylchitosane und deren Salze; N-Hydroxyalkylchitosanalkylether; N-Hydroxyalkylchitosanbenzylether; Copolymer aus Vinylcaprolactam, Vinylpyrrolidon und Dimethylaminoethylmethacrylat; Copolymeren aus Vinylpyrrolidon und Dimethylaminoethylmethacrylat, Copolymeren aus Vinylpyrrolidon, Vinylcaprolactam und Dimethylaminopropylacrylamid; Poly- oder Oligoestern, aufgebaut aus mindestens einer ersten Monomerart, die ausgewählt ist aus mit mindestens einer quaternären Ammoniumgruppe substituierten Hydroxysäure;
- zwitterionischen und/oder amphoteren Polymeren, ausgewählt aus Copolymeren aus Octylacrylamid, Acrylsäure, Butylaminoethylmethacrylat, Methylmethacrylat und Hydroxypropylmethacrylat; Copolymeren aus Laurylacrylat, Stearylacrylat, Ethylaminoxidmethacrylat und mindestens einem Monomeren ausgewählt aus Acrylsäure, Methacrylsäure, Acrylsäureestern und Methacrylsäureestern; Copolymeren aus Methacryloylethylbetain und mindestens einem Monomeren ausgewählt aus Methacrylsäure und Methacrylsäureestern; Copolymeren aus Acrylsäure, Methylacrylat und Methacrylamidopropyltrimethylammoniumchlorid; Oligomeren oder Polymeren, herstellbar aus quaternären Crotonbetainen oder quaternären Crotonbetainestern.;
- nichtionischen Polymeren, ausgewählt aus Polyvinylpyrrolidon, Polyvinylcaprolactam, Vinylpyrrolidon/Vinylacetat Copolymeren, Polyvinylalkohol, Isobutylen/Ethylmaleimid/Hydroxyethylmaleimid Copolymer; Copolymeren aus Vinylpyrrolidon, Vinylacetat und Vinylpropionat; Terpolymere aus Vinylpyrrolidon, Methacrylamid und Vinylimidazol.

10. Gel nach einem der vorhergehenden Ansprüche, **dadurch gekennzeichnet, dass** es optisch klar ist.

11. Gel nach einem der vorhergehenden Ansprüche, **dadurch gekennzeichnet, dass** es mindestens ein Parfümöl in optisch klar einemulgierter Form enthält.

12. Gel nach einem der vorhergehenden Ansprüche, **dadurch gekennzeichnet, dass** es in Form eines Flüssiggels oder in Form eines Sprühgels, welches mit einer mechanischen Vorrichtung versprüht wird, vorliegt.

13. Gel nach einem der vorhergehenden Ansprüche, **dadurch gekennzeichnet, dass** es einen Gehalt aufweist an
(A) 0,5 bis 10 Gew.% mindestens eines Gelbildner, ausgewählt aus Acrylat/C10-30 Alkylacrylat Crosspolymeren, Carbomeren, Xanthangum und Acrylat/Ceteth-20 Itaconat Copolymer;
(B) 3 bis 15 Gew.% mindestens eines UV-Filters, ausgewählt aus organischen Verbindungen mit mindestens einer Benzimidazoleinheit und mindestens einer Sulfonsäuregruppe und deren Salzen und
(C) 0,1 bis 15 Gew.% mindestens eines haarfestigenden Polymers, ausgewählt aus anionischen und nichtionischen Polymeren.
